# DEMANDE DE BREVET EUROPEEN

(11) **EP 1 836 994 A1**
(43) Date de publication de la demande: **26.09.2007**
(21) Numéro de dépôt: 07103609.9
(22) Date de dépôt: 06.03.2007
(51) Int. Cl.: A61F 2/00

(54) **Dispositif chirurgical pour la formation par le praticien d'au moins un implant**

(30) Priorité: 21.03.2006 FR 0602460
(71) Demandeur: Cousin Biotech, 59117 Wervicq Sud (FR)
(72) Inventeur: Molitor, Audrey, 59000 Lille (FR); Charret, Mathieu, 59236 Frelinghien (FR)
(74) Mandataire: Hennion, Jean-Claude

(57) **Abrégé**

Le praticien, avant l'introduction d'un implant, en rectifie le contour selon la morphologie de la zone d'intervention chez le patient et/ou l'état de la pathologie à traiter. Le dispositif chirurgical de la présente invention permet la formation par le praticien d'au moins un implant ayant une configuration donnée et étant dans un matériau biocompatible déterminé, comprenant :
a) un panneau (4) dans ledit matériau et dont au moins une dimension est supérieure à celle correspondante de l'implant,
b) une première feuille (2) comportant le tracé (5,6) d'au moins le contour de l'implant,

et en ce que ladite première feuille (2) est placée sur une face dudit panneau (4) et associée à celui-ci de telle sorte que le praticien peut former l'implant en découpant l'ensemble constitué par la première feuille (2) et le panneau (4) associés en suivant ledit tracé (5,6).

Ledit tracé (5,6) peut comporter plusieurs jeux de lignes de découpe (12,13,14,15,20,21) délimitant plusieurs contours possibles de l'implant et ladite première feuille (2) peut comporter plusieurs tracés (5,6) de contour d'implants correspondant aux traitements de pathologies différentes.

## Description

La présente invention a pour objet un dispositif chirurgical pour la formation par le praticien d'au moins un implant de configuration déterminée.

Lors d'une intervention chirurgicale, notamment en chirurgie viscérale et urogynécologique, il est connu que le praticien, avant l'introduction d'un implant en rectifie le contour selon la morphologie de la zone d'intervention chez le patient et/ou l'état de la pathologie à traiter.

Le praticien dispose habituellement d'un implant déjà découpé selon une configuration prédéterminée adapté de façon générale à la pathologie. Il est donc d'une part limité au contour arrêté par l'implant et à la surface disponible de celui-ci et d'autre part n'est pas aidé dans sa mise en forme de l'implant - ayant souvent une configuration complexe - lui faisant ainsi perdre un temps précieux et prendre le risque de ne pas découper la forme finale de l'implant souhaitée.

En outre, pour répondre aux besoins des patients, les implants sont commercialisés séparément selon des configurations prédéfinies pour une même pathologie, ce qui augmente la gamme commerciale d'implants à fournir et à stocker chez les distributeurs ou dans les hôpitaux.

La présente invention a notamment pour but de pallier aux inconvénients précités en proposant un dispositif chirurgical pour la formation par le praticien d'au moins un implant ayant une configuration donnée et étant dans un matériau biocompatible déterminé, caractérisé en ce qu'il comprend :
- un panneau dans le dit matériau et dont au moins une dimension est supérieure à celle correspondante de l'implant,
- une première feuille comportant le tracé d'au moins le contour de l'implant,
et en ce que la dite première feuille est placée sur une face dudit panneau et associée à celui-ci de telle sorte que le praticien peut former l'implant en découpant l'ensemble constitué par la première feuille et le panneau associés en suivant le dit tracé.

Les dimensions du panneau étant supérieures ou égales à celles de l'implant, le praticien dispose de suffisamment dudit matériau pour mettre en forme à son gré l'implant. En outre, le praticien est guidé dans sa découpe du dit ensemble en suivant le tracé du contour de l'implant lui économisant ainsi un temps précieux et des erreurs de découpe irrémédiables.

Le panneau dans lequel est découpé l'implant est généralement plan. Il peut cependant être en trois dimensions : par exemple un panneau préformé et ondulé. L'implant obtenu a donc une configuration plane ou tridimensionnelle.

Le panneau est dans un matériau biocompatible, soit dans un matériau textile du type tissé, tricoté ou nontissé, par exemple un tricot en polypropylène ou polyester ou polyamide ou acide polylactique de forme L ou D; soit dans une feuille de polymère, par exemple une feuille calandrée de silicone.

La première feuille comportant le tracé, est placée sur une face du panneau. De préférence, elle est dans un matériau faisant apparaître lisiblement le tracé, et permettant aussi de visualiser le panneau, notamment il s'agit d'une feuille opaque ou transparente ou translucide. Le tracé d'au moins le contour de l'implant est réalisé de préférence avec une encre bio-compatible.

Dans une variante de réalisation, la première feuille est l'une des deux faces d'une enveloppe contenant ledit panneau. L'autre face de l'enveloppe, opposée à la première feuille comportant le tracé d'au moins le contour de l'implant, est appelée seconde feuille, et est choisie dans un matériau qui peut être le même que celui dans lequel est formée la première feuille ou différent de celui-ci. C'est l'ensemble constitué par la première, la seconde feuille et le panneau qui est découpé par le praticien. Le panneau est associé à la première feuille du fait qu'il est contenu dans la dite enveloppe quand bien même il est disposé librement à l'intérieur de celle-ci.

Dans une variante de réalisation, la première et la seconde feuille, formant les deux faces de l'enveloppe, sont associées sur tout ou partie de leur périphérie autour du panneau par soudure hautes fréquences, par soudure ultrasons, par thermosoudure, par collage ou tout autre moyen équivalent.

De préférence, les première et seconde feuilles, formant les deux faces de l'enveloppe, sont rectangulaires et associées sur deux côtés et éventuellement un troisième côté afin d'une part, d'apporter un maintien latéral lors de la découpe et ainsi faciliter la mise en forme de l'implant et d'autre part de laisser au moins une ouverture, formée dans ce cas selon le côté non associé, au travers de laquelle le panneau peut dépasser ou être accessible de sorte que un ou plusieurs liens puissent être fixés au matériau du panneau et sortir par la dite ouverture, hors de l'enveloppe leur évitant ainsi d'être malencontreusement coupés par le praticien lors de l'utilisation de l'outil de découpe. Ces liens sont enfilés par le praticien sur des instruments lui permettant l'introduction et la mise en place de l'implant dans la zone d'intervention chirurgicale.

Les dites première et seconde feuille, rectangulaires, peuvent également être soudées sur toute leur périphérie et contenir librement le dit panneau. Le maintien est amélioré puisque tous les côtés sont soudés.

Dans une variante de réalisation, la première feuille est soudée sur tout ou partie de sa périphérie autour du dit panneau par soudure hautes fréquences, par soudure ultrasons, par thermosoudure ou par collage. De préférence, ladite première feuille est soudée sur trois côtés audit panneau. On retrouve les mêmes avantages que dans la variante précédente, lorsque l'enveloppe contient le panneau à savoir le maintien latéral et l'éventuelle fixation de liens.

Dans cette variante, le panneau est indissociable de ladite première feuille contrairement à la variante précédente où le panneau étant disposé librement à l'intérieur de ladite enveloppe, il peut être remplacé par un autre panneau pris par exemple dans un matériau différent.

Dans une variante de réalisation, la dite première feuille est placée sur une face du dit panneau et associée à celui-ci au moyen d'un adhésif repositionnable biocompatible. L'adhésif recouvre généralement tout ou partie de la surface de l'une des faces de la dite première feuille. On dit qu'un adhésif est repositionnable lorsqu'il peut être décollé du support sur lequel il est appliqué, sans préjudice pour le dit support, et être éventuellement repositionné, sur le même ou un autre support, son adhérence étant suffisante pour lui assurer une bonne solidarisation avec le dit support.

Avantageusement, durant la découpe de l'ensemble constitué par la première feuille et le panneau, la première feuille reste associée au panneau. Le praticien peut décoller la première feuille et la coller en la positionnant différemment sur une face du dit panneau autant de fois que nécessaire. Après découpe du dit ensemble en suivant le tracé, le praticien termine l'implant en décollant la première feuille du panneau.

Bien sûr, l'ensemble formé par la première feuille associée directement au dit panneau ou à une deuxième feuille avec laquelle elle forme une enveloppe contenant librement le dit panneau, n'est pas limité à une forme rectangulaire et peut prendre n'importe quelle forme selon l'application visée.

Dans une variante de réalisation, le tracé comporte plusieurs jeux de lignes de découpe délimitant plusieurs contours possibles de l'implant. Le praticien est ainsi guidé sur des variantes de configuration pour un même implant, adapté à un type de pathologie déterminée, ce qui lui fait gagner du temps, un certain confort en lui évitant des erreurs de découpe et économiser la fourniture séparée de chaque variante d'implant.

Dans une variante de réalisation, ladite première feuille comporte plusieurs tracés de contour d'implants correspondant aux traitements de pathologies différentes. Par exemple, ladite première feuille peut comporter deux tracés avec, pour chacun d'eux, plusieurs jeux de lignes de découpe, chaque tracé étant destiné à former un implant pour le traitement d'une pathologie donnée. Cette disposition permet avantageusement d'économiser la fourniture séparée d'implant selon chaque pathologie que ce soit au niveau de la production (découpe par les opérateurs, emballage, stérilisation, ...etc.) ou lors de la distribution commerciale.

Le dispositif chirurgical de la présente invention, une fois fabriqué, est ensuite emballé dans deux sachets, appelés également double sachet, puis stérilisé, par exemples à l'oxyde d'éthylène ou aux rayons gamma .

Dans une variante de réalisation, le dispositif chirurgical comporte au moins un lien solidaire du panneau, destiné à servir d'attache pour l'implant.

Ladite attache facilite pour le praticien l'introduction et la mise en place de l'implant sur la zone du patient à traiter en s'enfilant facilement sur un instrument type introducteur. Le dit lien se présente par exemple sous forme d'une boucle ou peut être une agrafe.

Ledit lien est de préférence solidarisé à la partie du panneau dépassant ou accessible depuis l'ouverture formée par une partie non soudée ou collée de l'enveloppe ou de la première feuille, de façon à ce que le praticien ne soit pas gêné ou risque de le couper lors de la découpe de l'implant. Pour le cas où l'une des faces de la première feuille est revêtue sur tout ou partie de sa surface d'un adhésif repositionnable, de préférence la dite face est associée à une face du dit panneau de façon à ne pas recouvrir le dit lien et le laisser libre.

Dans une variante de réalisation, ledit tracé correspond au contour d'un implant destiné au traitement d'une rectocèle, d'une cystocèle, d'une hernie ou d'une éventration.

Dans variante de réalisation, le dispositif chirurgical de manière caractéristique comprend un panneau dépassant de la première feuille, notamment selon des prolongements destinés à former des pattes d'attache de l'implant.

Les dites pattes d'attache peuvent ainsi sortir de la première feuille notamment selon une longueur supérieure à la longueur du côté de la première feuille au travers duquel elles dépassent.

La présente invention sera mieux comprise à la lecture de deux exemples de dispositif chirurgical, cités à titre non limitatif, et illustrés par les figures ci-après :
- la figure 1 est une vue schématique en plan, de dessus, d'un premier exemple de dispositif chirurgical selon la présente invention ;
- la figure 2 est une vue schématique en plan, de dessus, d'un deuxième exemple de dispositif chirurgical selon la présente invention ;

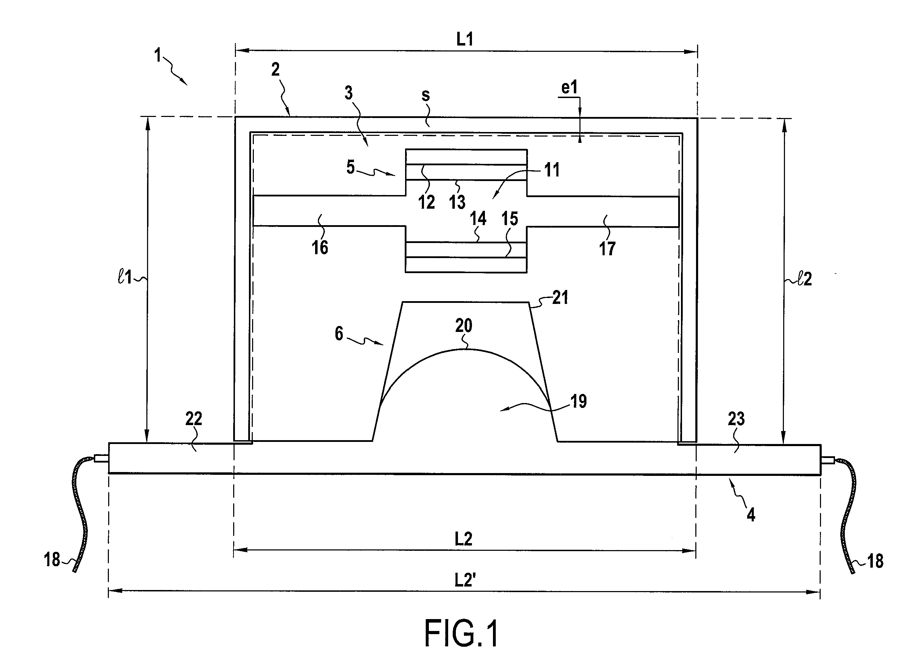

A la figure 1, le dispositif chirurgical 1 selon la présente invention se présente sous la forme d'une enveloppe 2, rectangulaire, laquelle comprend un panneau 4 plan disposé librement à l'intérieur de celle-ci. L'enveloppe 2 a deux faces à savoir une première feuille 3 et une seconde feuille non visible sur la figure 1 puisqu'elle a les mêmes dimensions que la première feuille 3 et se trouve sur l'envers de l'enveloppe 2. La première feuille 3 et la seconde feuille formant l'enveloppe, sont thermo soudées sur deux petits côtés ℓ1 et ℓ2 et leur grand côté L1, selon la ligne de soudure s et sur une largeur e1. De préférence e1 vaut quelques millimètres, plus particulièrement e1 vaut 5 millimètres. Le panneau 4 dépasse du grand côté non soudé L2 selon deux prolongements 22 et 23, sur une longueur L2' supérieure à la longueur L2. Ces prolongements 22,23 font office de pattes d'attache à l'implant sur les tissus et/ou organes du patient.

La première feuille 3 comporte, imprimé sur l'une de ses faces, mais visibles extérieurement, deux tracés de contour d'implants correspondant au traitement de deux pathologies différentes. La première pathologie est la rectocèle et correspond au premier tracé 5. La seconde pathologie est la cystocèle et correspond au second tracé 6. Chaque tracé 5,6 comporte plusieurs jeux de lignes de découpe afin de proposer au praticien différentes variantes de configuration plane pour mettre en forme l'implant. Lors de l'utilisation du dispositif chirurgical 1, le praticien selon la pathologie à traiter : rectocèle ou cystocèle, et la configuration qu'il souhaite donner à l'implant, découpe l'ensemble formé de la première feuille 3 et la seconde feuille associées au panneau 4 en suivant le contour des lignes de découpe choisies et forme ainsi un implant de configuration adaptée à la pathologie et au patient, prêt à l'emploi.

De préférence, le praticien commence la découpe dudit ensemble à partir d'un petit côté ℓ1 et/ou ℓ2, le grand côté L1 apportant ainsi un maintien latéral préservant l'association du panneau 4 avec l'enveloppe 2 tout du moins en début de découpe. Le praticien réalise la découpe du dit l'ensemble à l'aide de ciseaux classiques ou électriques, ou autre moyen équivalent.

Une fois la découpe dudit ensemble réalisée sur tout le contour de la configuration choisie, le panneau 4 étant disposé librement dans l'enveloppe 2, l'implant de configuration arrêtée par le praticien est dégagé dudit ensemble et prêt à être introduit et placé sur la zone du patient à traiter.

Les deux tracés 5,6 représentés à la figure 1 correspondent à des implants ayant une portion centrale ; respectivement 11 pour la rectocèle 5 et 19 pour la cystocèle 6, dont les dimensions peuvent ajustées selon des lignes de découpe. Dans le cas du premier tracé 5 pour la rectocèle, la superficie de la portion 11 peut être ajustée selon les lignes de découpe 12, 13, 14 et 15. De plus, le premier tracé 5 délimite deux bandes 16,17 dans le prolongement l'une de l'autre qui s'étendent à partir de la portion centrale 11. Dans le cas du second tracé 6 pour la cystocèle, le praticien peut choisir une forme arrondie pour la portion centrale 19 en suivant la ligne de découpe 20 ou une forme en trapèze en suivant la ligne de découpe 21 selon les dimensions de la zone d'intervention chirurgicale.

Dans le cas du tracé 6 pour la cystocèle, le panneau 4 dépasse hors de l'enveloppe 2 sur le second grand côté L2 et comporte deux prolongements latéraux 22,23 qui transversalement s'étendent de la portion centrale 19, au-delà dudit second grand côté L2. Deux liens 18 sont sertis aux deux extrémités desdits prolongements et forment des attaches en forme de boucles pouvant être passées par le praticien sur des instruments du type introducteur l'aidant à introduire et mettre en place l'implant chez le patient. Dans cet exemple de réalisation, les liens 18 sont des tresses.

De préférence, le panneau 4 est dans un matériau textile biocompatible et plus particulièrement dans un tricot en polypropylène. Etant donné que le panneau 4 est disposé librement dans l'enveloppe, il est possible de le remplacer par un panneau, ayant les mêmes dimensions, pris dans un matériau biocompatible différent par exemple un tricot en polyester imprégné de silicone implantable ce qui permet non seulement de proposer au praticien différentes configurations pour la mise en forme d'un implant grâce aux tracés, lesquels comportent éventuellement des lignes de découpe, portés sur l'une des faces de l'enveloppe mais également le choix du matériau le constituant grâce à la capacité de substitution du panneau dans ladite enveloppe.

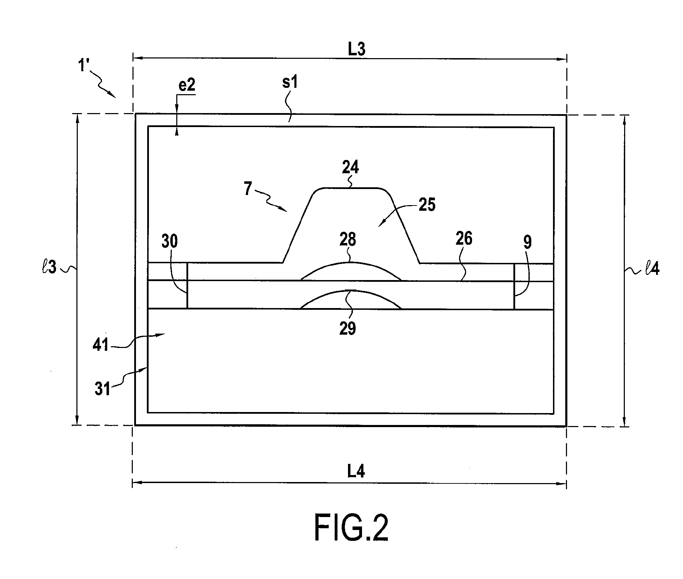

Le second exemple de dispositif chirurgical 1' selon la présente invention, représenté à la figure 2, est plus simple de réalisation que le dispositif 1. Il comprend uniquement une première feuille 31 et un panneau 41 rectangulaires. La première feuille 31 comporte un seul tracé 7 de contour d'implant correspondant au traitement d'une pathologie. Dans ce cas particulier, il s'agit de la cystocèle. Le tracé 7 porté sur la première feuille 31 comporte de nombreuses lignes de découpe : 9, 24, 26, 28, 29, 30. Le praticien peut ajuster le traitement de la cystocèle en découpant selon la ligne de découpe 28 ou 29, laquelle crée une encoche dans la portion 25, ce qui lui permet dans le cas de cette pathologie de laisser passer le col vésical et ainsi de ne traiter que le prolapsus. La première feuille 31 est collée sur toute sa périphérie, constituée par deux petits côtés ℓ3 et ℓ4 et les deux grands côtés L3 et L4, directement sur le panneau 41 sur une largeur e2 selon la ligne de soudure s1. Il n'y pas de lien fixé sur le panneau 41. Dans ce cas, contrairement au premier exemple de réalisation, il n'est pas possible de changer le panneau 41 une fois celui-ci associé à la première feuille. Le praticien forme l'implant en découpant le panneau 41 et la première feuille 31 selon le tracé 7 à partir des lignes de découpe qu'il a choisies. L'implant se détache facilement du panneau 41 et de la première feuille 31 puisque la soudure n'a lieu que sur une largeur e2. Il dispose également d'un maintien latéral, tout du moins en début de découpe. En effet, lorsque le praticien commence la découpe à partir de l'un des petits côtés, les grands côtés L3 et L4 maintiennent latéralement le panneau 41 et la première feuille 31, et inversement lorsque la découpe débute à partir d'une des longueurs L3 ou L4.

## Revendications

1. Dispositif chirurgical pour la formation par la praticien d'au moins un implant ayant une configuration donnée et étant dans un matériau biocompatible déterminé, **caractérisé en ce qu'**il comprend :
a) un panneau dans ledit matériau et dont au moins une dimension est supérieure à celle correspondante de l'implant,
b) une première feuille, qui n'est pas destinée à être implantée et qui comporte le tracé d'au moins le contour de l'implant,
et **en ce que** ladite première feuille est placée sur une face dudit panneau et associée à celui-ci de telle sorte que le praticien forme l'implant en découpant l'ensemble constitué par la première feuille et le panneau associés en suivant ledit tracé et en retirant la portion découpée de ladite première feuille.

2. Dispositif selon la revendication 1, **caractérisé en ce que** la dite première feuille est l'une des deux faces d'une enveloppe contenant le dit panneau.

3. Dispositif chirurgical selon la revendication 2, **caractérisé en ce que** les deux faces de l'enveloppe sont associées sur tout ou partie de leur périphérie autour du panneau par soudure hautes fréquences, par soudure ultrasons, par thermo soudure, ou par collage.

4. Dispositif chirurgical selon la revendication 1, **caractérisé en ce que** la dite première feuille est soudée sur tout ou partie de sa périphérie autour du dit panneau par soudure hautes fréquences, par soudure ultrasons, par thermo soudure, ou par collage.

5. Dispositif chirurgical selon la revendication 1, **caractérisé en ce que** la dite première feuille est placée sur une face du dit panneau et associée à celui-ci au moyen d'un adhésif repositionnable biocompatible.

6. Dispositif chirurgical selon l'une quelconque des revendications précédentes, **caractérisé en ce que** le dit tracé comporte plusieurs jeux de lignes de découpe délimitant plusieurs contours possibles de l'implant.

7. Dispositif chirurgical selon l'une quelconque des revendications précédentes, **caractérisé en ce que** la dite première feuille comporte plusieurs tracés de contour d'implants correspondant aux traitements de pathologies différentes.

8. Dispositif chirurgical selon l'une quelconque des revendications précédentes, **caractérisé en ce qu'**il comporte au moins un lien solidaire du panneau, destiné à servir d'attache pour l'implant.

9. Dispositif chirurgical selon l'une quelconque des revendications précédentes, **caractérisé en ce que** le dit tracé correspond au contour d'un implant destiné au traitement d'une rectocèle, d'une cystocèle, d'une hernie ou d'une éventration.

10. Dispositif chirurgical selon l'une quelconque des revendications précédentes, **caractérisé en ce que** ledit panneau dépasse de la première feuille, notamment selon des prolongements destinés à former des pattes d'attache de l'implant.
